# EUROPEAN PATENT SPECIFICATION

(11) **EP 2 541 247 B1**
(45) Date of publication and mention of the grant of the patent: **16.09.2015**
(21) Application number: 11747426.2
(22) Date of filing: 24.02.2011
(51) Int. Cl.: G01N 33/53, G01N 33/12, G01N 33/543

(54) **METHOD FOR DETECTING RAW PORK AND DETECTION KIT THEREFOR**
VERFAHREN ZUM ERKENNEN VON ROHEM SCHWEINEFLEISCH UND ERKENNUNGSKIT DAFÜR
PROCÉDÉ DE DÉTECTION DE PORC CRU ET KIT DE DÉTECTION POUR CE PROCÉDÉ

(30) Priority: 25.02.2010 JP 2010039631
(43) Date of publication of application: 02.01.2013
(73) Proprietor: Tanaka Kikinzoku Kogyo K.K., Chiyoda-ku Tokyo 100-6422 (JP)
(72) Inventor: SAKAKIBARA Yuhiro, Hiratsuka-shi Kanagawa 254-0076 (JP); MOCHIDUKI Kazuyoshi, Hiratsuka-shi Kanagawa 254-0076 (JP); SHIBAI Yusuke, Hiratsuka-shi Kanagawa 254-0076 (JP); IWAMOTO Hisahiko, Hiratsuka-shi Kanagawa 254-0076 (JP)
(74) Representative: Schubert, Klemens
(86) International application number: PCT/JP2011/054073
(87) International publication number: WO 2011/105466

(56) References cited:
- CN-Y- 2 911 690
- JP-A- 8 098 644
- JP-B1- 4 382 866
- Sonia Myers ET AL: "Immunological detection of adulteration of ground meats by meats of other origins", Biotechnology Techniques, 1 July 1997 (1997-07-01), pages 533-535, XP055071740, Retrieved from the Internet: URL:http://rd.springer.com/content/pdf/10. 1023/A:1018426403085.pdf [retrieved on 2013-07-17]
- "Labelled goat anti pig IgG Antibodies", Molecular probes product information, 20 February 2001 (2001-02-20), pages 1-2, XP055071742, Retrieved from the Internet: URL:http://probes.invitrogen.com/media/pis /mp21232.pdf [retrieved on 2013-07-17]
- KOICHI WATANABE ET AL.: 'Shokuniku.Shokuniku Seihin no Soshikigaku Kohen' SHOKUNIKU NO KAGAKU vol. 46, no. 1, 2005, pages 1 - 9

## Description

### Technical Field

The present invention relates to a method of detecting (raw) pork in non-heated food and a detection kit therefor. Furthermore, the present invention relates to a reagent for immunochromatography that detects (raw) pork in non-heated food using an antibody obtained by immunizing an animal with immunoglobulin G in porcine serum and specifically recognizing the immunoglobulin G.

### Background Art

Since the EU Scientific Steering Committee stated their report on a risk of infection of bovine infectious diseases, such as bovine spongiform encephalopathy (BSE), to human being, use of meat-and-bone meal of BSE-infected cows as livestock feed has been acknowledged as a problem. In addition, examination for disguised food, such as fake brand for place branded beef, has also been the subject of attention.

At the same time, in patients with food allergy, various allergic symptoms, such as asthma, dermatitis, gastrointestinal dysfunction, and anaphylactic shock, are caused by food allergens contained in food. In some cases, the symptoms are serious. There is a tendency of an increase in number of food allergy patients, and consumers are highly interested in food safety.

As typical examples of food containing food allergens, generally well known are grain (e.g., buckwheat and wheat), eggs, meat (e.g., beef, pork, and chicken), fishes (e.g., mackerel and sardine), milk, shellfishes (e.g., crab), mollusks, beans (e.g., peanut and soybean), fruits (e.g., mango), and vegetables (e.g., garlic). Such food allergy-inducing food contains food allergy-inducing ingredients such as gluten, gelatin, casein, ovalbumin, ovomucoid, lysozyme, α-lactoalbumin, or β-lactoglobulin. Processed food contains many kinds of food and many kinds of food allergens, but currently there is no way to conveniently detect them.

In addition, in processed food (for example, by heating, pressurizing, enzyme treatment, freezing, drying, or salting), the protein ingredients in the food are denatured in their molecular structures or molecularly modified by the processing. Therefore, in order to conveniently detect these ingredients in processed food, it is important to use appropriate antigens and epitopes for corresponding processed ingredients and non-processed raw ingredients, and researches and studies therefor have been conducted.

For example, as a detection reagent for detecting a material in a sample, such as canned food or livestock feed, derived from animal tissue, represented by cow, hog, and chicken, heat-treated at a high temperature such as a temperature of higher than 100°C, it has been found that an antibody produced by an animal immunized with heat-denatured serum albumin protein as an immunogen specifically recognizes heat-denatured serum albumin and does not have a cross-reactivity with non-heated serum albumin, and proposed is a method (including immunochromatography) for detecting the presence of a heat-treated material derived from animal tissue in a sample (see Patent Literatures 1 and 2).

In addition, an IgE (immunoglobulin E) antibody obtained by immunizing an animal with a mixture (protein) of food allergens composed of non-denatured and/or denatured materials and a method of detecting a food allergen and food allergy-inducing food with the antibody are known (see Patent Literature 3).

Furthermore, it is known that pork in heat-treated food can be detected by ELISA (an immunological measurement method) using a monoclonal antibody produced by a deposited specific cell line (a cell line obtained by immunizing an animal with a specific protein in heated pork) (see Patent Literature 4).

Sonia Myers et al. ("Immunological detection of adulteration of ground meats by meats of other origins." 1997; Biotechnology Techniques , 533-535) used a rabbit anti pig IgG antibody bond to a cloth to bind pork IgG and thus highlight its presence in a sample suspected of pork contamination.

Furthermore on the product sheet from molecular probes ("Labelled goat anti pig IgG Antibodies." 2001, Molecular probes product information ; 1-2) consumable available goat anti pig IgG antibodies are described.

In addition, in the document CN 2 911 690 Y a method for identifying a viral protein in pigs (PCV2) using an immuno-chromatographical test strip is described.

Since this detection process uses a monoclonal antibody produced by a specific cell line (a cell line obtained by immunizing an animal with a specific protein in heated pork), the detection is accurate. However, the preparation of the monoclonal antibody is expensive, and, therefore, there is a problem that the method is not suitable for applying to many patients in the medical field or to general consumers having different dietary cultures.

The present inventors have conducted intensive studies on immunochromatography for detecting raw pork in non-heated food using a polyclonal antibody instead of the monoclonal antibody. The results showed that when a rabbit-derived polyclonal antibody obtained by immunization of rabbit was used, reaction occurred even when a standard buffer only was used. Thus, non-specific reaction was strongly observed, and, therefore, it could not be used from the viewpoint of accuracy. Thus, it was observed that non-specific reaction still occurred when raw pork in non-heated food, the object to be detected in a sample, was detected by immunochromatography using a polyclonal antibody. Therefore, there still remained a problem that non-specific reaction could not be sufficiently inhibited.

As a way for inspecting mixing of other meat (e.g., beef, pork, or chicken) in minced meat, a cloth-based enzyme immunoassay (CEIA), an immunological method, is known. It is known that when mixing of pork is inspected, rabbit anti-porcine IgG-horseradish peroxidase (HRP)-labeled antibody is used as a labeled antibody (conjugate) for the inspection (see Non Patent Literature 1).

However, in the CEIA, it takes about 30 minutes for the test, and also a mixing concentration of 5% or more of other meat into beef is necessary to be detected. Thus, the method is low in detection sensitivity and is therefore insufficient practically.

The present inventors have developed studies for a purpose of applying the CEIA to immunochromatography that is more practical and simple and short in detection time. It was revealed that the use of rabbit anti-pork IgG as a detection antibody caused non-specific recognition not to allow the use. Thus, further studies were necessary.

Conventionally, as tests of meat, immunological methods such as CEIA and enzyme-linked immunosorbent assay (ELISA) and genetic methods such as PCR are known. The detection time of ELISA is shorter than that of PCR, but is considerably longer than that of immunochromatography. And also a detection apparatus is necessary. In addition, ELISA is low in specificity compared to PCR. PCR is high in specificity, but it takes about 3 hours for detection. In addition, it has a problem that equipment such as a gene amplification apparatus and a detection apparatus is necessary.

### Summary of Invention

### Technical Problem

It is an object of the present invention to provide an immunochromatographic detection process optimal for detecting by immunoassay raw pork in non-heated food with rapidity, high performance, and high sensitivity without causing non-specific reaction, by preparing a detection antibody for specifically recognizing raw pork contained in non-heated food by differentiating it from other meat, more specifically, to provide a convenient and high-accuracy immunochromatographic detection process for detecting raw pork in non-heated food using a polyclonal antibody that can differentiate raw pork contained in non-heated food from other meat as a detection antibody specifically recognizing immunoglobulin G of a specific protein and a detection kit therefor.

### Solution to Problem

The present invention relates to immunochromatographic detection of protein derived from raw pork in non-heat-processed food. The developer solution composition used in the present invention and the composition of chemical materials (other than biological materials) contained in the test kit are reagents widely used in conventional immunochromatography. For example, a developer solution is purified water or a buffer containing a nonionic surfactant (e.g., Tween 20 or Triton-100), sodium chloride, and a preservative (e.g., sodium azide).

The present inventors have used immunoglobulin G (hereinafter abbreviated to "IgG") that is a protein purified from porcine serum for preparing an antibody used in a detection system for non-heated food. The IgG may be commercially available one.

Accordingly, the detection antibody (biological material) used in immunochromatographic detection according to the present invention for detecting protein derived from raw pork in non-heated food is particularly suitable for a detection system for non-heated food. The detection antibody differs from not only those used in immunochromatographic detection systems for proteins derived from pork in heated food, but also from detection antibodies (biological materials) used in conventionally known immunological detection of proteins derived from pork in non-heated food. The present inventors have specified the detection antibody and have first completed the invention relating to a method of examining (detecting) the presence or absence of raw pork in non-heated food by immunochromatography and an examination (detection) kit.

In the detection system for non-heated food according to the present invention, for example, a polyclonal antibody specifically recognizing IgG of a specific protein contained in raw pork is preferably used as a detection antibody.

The present invention provides the following (a) to (f), an immunochromatographic detection process, an antibody used therefor, a detection apparatus, and a detection kit used therefor:
(a) A first aspect of the present invention relates to an immunochromatographic detection process of a protein derived from raw pork using a polyclonal antibody as a detection antibody, wherein the polyclonal antibody is obtained by immunizing a goat with porcine immunoglobulin G and said antibody specifically recognizes immunoglobulin G contained in raw pork.
(b) A second aspect of the present invention relates to the immunochromatographic detection process according to the aspect (a), wherein the detection antibody is goat anti-porcine immunoglobulin G antibody.
(c) A third aspect of the present invention relates to the immunochromatographic detection process of a protein derived from raw pork according to aspect (a), wherein the said process is performed by means of an apparatus whereas said apparatus includes a labeled material-holding portion holding a polyclonal antibody obtained by immunizing a goat with porcine immunoglobulin G, specifically recognizing immunoglobulin G contained in raw pork, and labeled with a labeling material; and a detection portion where a goat polyclonal antibody specifically recognizing immunoglobulin G contained in raw pork is immobilized.
(d) A fourth aspect of the present invention relates to the immunochromatographic detection process according to the aspect (c), wherein the apparatus being substantially composed of a sample application portion, a labeled material-holding portion, a chromatography medium, a detection portion, and an absorbing portion.
(e) A fifth aspect of the present invention relates to the immunochromatographic detection process for detecting a protein derived from raw pork, according to aspect (a), using a kit being substantially composed of a sample application portion, a labeled material-holding portion, a chromatography medium, a detection portion, and an absorbing portion, wherein the labeled material-holding portion holds a polyclonal antibody obtained by immunizing a goat with porcine immunoglobulin G, specifically recognizing immunoglobulin G contained in raw pork, and labeled with a labeling material; and the detection portion has a goat polyclonal antibody specifically recognizing immunoglobulin G contained in raw pork immobilized thereon.
(f) A sixth aspect of the present invention relates to the immunochromatographic detection process according to the aspect (e), using a kit wherein the labeled antibody and the antibody immobilized on the detection portion are obtained by immunizing goats by the same method.

### Advantageous Effects of Invention

In the present invention, since an antibody obtained by immunizing a goat or a rabbit (in the case of rabbit antibodies, preliminary processing is necessary) with IgG, a specific protein, contained in raw pork is used as the detection antibody (biological material), even though the antibody is polyclonal, there are no cross reaction not only with proteins derived from plants such as soybean but also with proteins derived from animals other than hog, such as cow, chicken, and sheep, and no reduction in sensitivity, and it is possible to accurately and easily determine the result of immunochromatographic detection of a protein derived from raw pork in non-heated food.

The immunochromatographic detection of a protein derived from raw pork according to the present invention can also be conveniently and inexpensively used for detecting a protein derived from raw pork contained not only in non-heated food but also in health food, medicines, livestock feed, pet food, and so on that have been subjected to processing treatment (e.g., pressurizing, shaping, or drying) that does not cause protein denaturation. Therefore, the present invention can contribute to inexpensively, conveniently, and accurately solve various problems relating to meat so as to deal with the problems of patients to whom pork causes allergy reaction or of conventional wisdom depending on the reasons, for example, of not eating pork in terms of tastes or dietary cultures, that is, dietary habit.

Since the immunochromatographic detection kit according to the present invention is a simpler device compared to PCR and ELISA methods, the operation is easy, and a protein derived from raw port in non-heated food can be rapidly and accurately measured. In addition, since the device can be supplied at a very low price, the kit has an excellent advantage in which the device can become common and appropriately contribute to the need of general consumers.

### Brief Description of Drawings

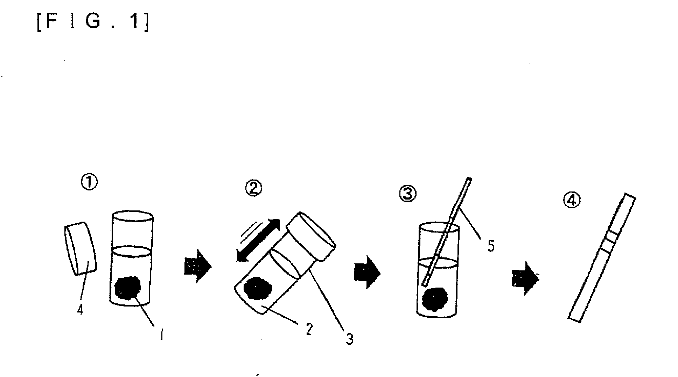

Fig. 1 is a schematic diagram illustrating the use of an immunochromatographic detection kit according to the present invention. Description of Embodiments

The present invention will be described in detail below.

The present invention has been accomplished by focusing on a protein, in particular, immunoglobulin G (hereinafter also referred to as "IgG") contained in raw pork as means of specifically recognizing raw pork contained in non-heated food by differentiating it from other meat by immunoassay, and based on the finding of a polyclonal antibody for immunochromatographic detection of IgG, effectively functioning as an antibody that recognizes the protein in immunochromatography. As an antibody for detecting the IgG, it is conventionally known that a labeled antibody (conjugate) labeled with an enzyme is effective in CEIA, but, for example, the performance of differentiation, sensitivity, and the necessary time are unsatisfactory.

As antibodies for detecting IgG, various antibodies having specificity, for example, chicken-derived monoclonal (or polyclonal) antibodies, which are those of a typical bird having exhaustive immunocompetence and also being excellent in antibody producibility, and mouse-derived monoclonal (or polyclonal) antibodies, horse-derived monoclonal (or polyclonal) antibodies, goat-derived monoclonal (or polyclonal) antibodies, and rabbit-derived monoclonal (or polyclonal) antibodies are proposed. However, the inventors have conducted intensive studies on these monoclonal (or polyclonal) antibodies and have found that polyclonal antibodies obtained by immunizing a goat or a rabbit (in the case of rabbit antibodies, preliminary processing is necessary) with IgG are suitable as detection antibodies that can show high performance and be supplied most conveniently and inexpensively.

Furthermore, the antigen-binding fragments of goat-derived polyclonal antibody and rabbit-derived polyclonal antibody can detect IgG of raw pork in non-heated food with rapidity, high performance, and high sensitivity without causing non-specific reaction and are therefore very effective.

Accordingly, the identification performance of the antigen-binding fragments of the goat-derived polyclonal antibody and rabbit-derived polyclonal antibody is optimal for immunochromatographic detection.

More specifically, a most preferred embodiment can provide a convenient and high-accuracy immunochromatographic detection process using polyclonal antibodies composed of the antigen-binding fragment of the goat-derived polyclonal antibody or the rabbit-derived polyclonal antibody as a detection antibody for specifically recognizing IgG, a specific protein, so that raw pork in non-heated food can be distinguished from other meat, and also can provide a detection kit therefor.

The goat-derived polyclonal antibody can be directly used as an antibody. However, in the case of the rabbit-derived polyclonal antibody, at least when it is used as a labeled detection antibody, an antigen-binding fragment obtained by cutting the Fc region having high lipophilicity with an enzyme can be directly used as an antibody for identification.

An embodiment of a test kit using the goat-derived polyclonal antibody or the rabbit-derived polyclonal antibody in detection of IgG of raw pork in non-heated food according to the present invention will be described below.

The composition of a developer solution and the composition of chemical components (other than biological materials) included in the test kit according to the present invention are the same as reagents widely used in conventional immunochromatography. As the reagents for immunochromatography, various chemical materials, such as a nonionic surfactant, a salt, a preservative, according to circumstances, a buffering agent, a chelating agent, can be used for achieving their functions and purposes.

Examples of the nonionic surfactant that can be contained in the immunochromatographic reagent composition of the present invention include polyoxyethylene alkyl ethers, polyoxyethylene/polyoxypropylene alkyl ethers, polyoxyethylene sorbitan fatty acid esters (trade name: "Tween" series), polyoxyethylene p-t-octylphenyl ethers (trade name: "Triton" series), polyoxyethylene p-t-nonylphenyl ethers (trade name: "Triton N" series), alkyl polyglucosides, fatty acid diethanolamide, and alkyl monoglyceryl ethers. The nonionic surfactants may be used alone or as a mixture of two or more.

The content of the nonionic surfactant in the immunochromatographic reagent composition of the present invention is in a range of 0.01 to 10% by weight, preferably in a range of 0.05 to 5% by weight to the immunochromatographic reagent composition. A content of less than 0.01 % by weight, for example, 0.005% by weight cannot give accurate determination. A content of less than 0.05% by weight is insufficient for inhibiting non-specific reaction, resulting in a tendency of having a slight difficulty in accurate determination. A content of higher than 10% by weight, e.g., 12% by weight or 18% by weight, is unnecessarily high, which does not advantageously affect inhibition of non-specific reaction and is also technologically meaningless and economically wasteful.

The nonionic surfactant can be optionally used together with, for example, another nonionic surfactant or ionic surfactant.

Examples of typical salt that can be contained in the immunochromatographic reagent composition, such as an extraction developer solution, of the present invention include sodium chloride, potassium chloride, calcium chloride, and magnesium chloride. Preferred is sodium chloride.

The concentration of the salt contained in the immunochromatographic reagent composition, such as an extraction developer solution, of the present invention is in a range of 0.02 to 2 M, preferably in a range of 0.05 to 1 M, and more preferably in a range of 0.1 to 0.5 M. A concentration of smaller than 0.02 M, for example, a low concentration of 0.002 M, is insufficient for extracting protein. A concentration of larger than 2 M, for example, a high concentration of 5 M or 10 M, is technologically meaningless and unnecessarily high to be economically wasteful.

As the salt contained in the immunochromatographic reagent composition of the present invention, not only one kind but also two or more kinds of salts can be used.

The buffering agent contained, according to need, in the immunochromatographic reagent composition, such as an extraction developer solution, of the present invention is not particularly limited as long as it can achieve the function (buffering function) without being significantly affected by a change in concentration due to addition of a sample or evaporation or dilution of the sample or contamination with a slight amount of foreign substances from the outside.

Examples of the buffering agent in the present invention include acetate buffers (acetic acid and sodium acetate), phosphate buffers (phosphoric acid and sodium phosphate), citrate buffers (citric acid and sodium citrate), borate buffers, Tris hydrochloride buffers (Tris(hydroxylmethyl)aminomethane and hydrochloric acid), TE buffers (Tris and ethylenediamine tetraacetic acid), TAE buffers (Tris, acetic acid, and ethylenediamine tetraacetic acid), TBE buffers (Tris, boric acid, and ethylenediamine tetraacetic acid), and HEPES buffer (2-[4-(2-hydroxyethyl)-1-piperazinyl]ethane sulfonic acid. These buffering agents may be used alone or in combination of two or more thereof. Preferred are acetate buffers, phosphate buffers, and Tris hydrochloride buffers, and more preferred are Tris hydrochloride buffers.

The concentration of the buffering agent contained, according to need, in the immunochromatographic reagent composition of the present invention is in a range of 0.01 to 250 mM, preferably in a range of 10 to 200 mM, and more preferably in a range of 30 to 180 mM. A concentration of smaller than 0.01 mM, for example, a concentration of 0.08 mM, is insufficient for buffering. A concentration of larger than 250 mM, for example, a concentration of 260 mM or 280 mM, is technologically meaningless and unnecessarily high to be economically wasteful.

As the buffering agent contained, according to need, in the immunochromatographic reagent composition of the present invention, not only one kind but also two or more kinds of buffering agents can be used.

The chelating agent contained, according to need, in the immunochromatographic reagent composition of the present invention is not particularly limited as long as it can function as a ligand having a plurality of coordination positions.

Examples of the chelating agent in the present invention include ethylenediamine, dipyridine, ethylenediamine tetraacetic acid (hereinafter referred to as "EDTA"), EDTA·2Na, EDTA3·Na, EDTA·4Na, EDTA derivatives (e.g., EDTA·2NH₄, EDTA·3K, EDTA·special amine salt), EDTA metal salts (e.g., EDTA·Ca·2Na), hydroxyethylethylenediamine triacetic acid (HEDTA) systems, dihydroxyethylethylenediamine diacetic acid (DHEDDA) systems, 1,3-propanediamine tetraacetic acid (1,3-PDTA) systems, diethylenetriamine pentaacetic acid (DTPA) systems, triethylenetetramine hexaacetic acid (TTHA) systems, nitrilotriacetic acid (NTA) systems, gluconic acid systems, hydroxyethylimino diacetic acid (HIMDA) systems, L-aspartic acid-N,N-diacetic acid (ASDA) systems, aminotrimethylenephosphonic acid (NTMP) systems, hydroxyethanephosphonic acid (HEDP) systems, tetrasodium 3-hydroxy-2,2'-iminodisuccinate, phenanthroline, porphyrin, and crown ether.

The concentration of the chelating agent contained, according to need, in the immunochromatographic reagent composition of the present invention is in a range of 0.01 to 10 mM, preferably in a range of 0.1 to 5 mM, and more preferably in a range of 0.5 to 2 mM. A concentration of smaller than 0.01 mM, for example, a low concentration of 0.008 mM, is insufficient for inhibiting non-specific reaction, resulting in inaccurate determination. A concentration of larger than 10 mM, for example, a high concentration of 12 mM or 18 mM, is unnecessarily high and is economically wasteful.

The immunochromatographic reagent composition of the present invention may further contain additives that are known to inhibit side reaction based on biological affinity or inhibit non-specific reaction, for example, proteins (e.g., bovine serum albumin, casein, or gelatin), polymers (e.g., polyethylene glycol, methylcellulose, polyvinylpyrrolidone, polyvinyl alcohol, or dextran), or ionic surfactants or polyanions (e.g., dextran sulfate, heparin, polystyrene sulfonic acid, or chondroitin sulfate) that enhance antigen-antibody reaction or inhibit non-specific reaction, and preservatives or antimicrobials. These additives may be used alone or in combination, and such a use is possible and effective without any restriction. Furthermore, one or more of these proteins, polymers, ionic surfactants or polyanions that enhance antigen-antibody reaction or inhibit non-specific reaction, and the preservatives or antimicrobials may be held on the mobile path of the mobile phase on a chromatographic medium constituting the stationary phase, and such a use is possible and effective without any restriction.

In an embodiment of using the immunochromatographic reagent composition of the present invention, the composition can be held in a sample pad (sample application portion) in an immunochromatography apparatus by applying the composition to the sample pad or immersing the sample pad in the composition and then drying the sample pad. In another embodiment, the immunochromatographic reagent composition in the present invention is held on a chromatography medium by providing an additive-holding portion at an appropriate place between an end of the sample application portion and an absorbing portion and letting the composition to be held there. For example, the application area of the composition may be near the sample application portion or the labeled material-holding portion or its vicinity. In particular, an embodiment in which the composition is held on only the sample application portion and/or labeled material-holding portion is preferred.

The method of using the immunochromatographic reagent composition of the present invention is not limited to the above-mentioned embodiments, and the composition may be used as an extraction developer solution or a diluent for samples. For example, the extraction developer solution of the present invention is ultrapure water or a buffer containing a nonionic surfactant (e.g., Tween 20 or Triton-100), sodium chloride, a preservative (e.g., sodium azide), etc., but may further contain the above-mentioned additives (e.g., bovine serum albumin, PVP, etc.) that are known to inhibit side reaction based on biological affinity or inhibit non-specific reaction.

As the extraction developer solution, usually, ultrapure water is used as a solvent, and a nonionic surfactant, protein, and a polymer, and, according to need, a buffer and a chelating agent are added to the solvent. The addition order is not particularly limited, and they may be added at once. When the composition is used as an extraction developer solution, the form of the extraction developer solution can be optimized depending on the state, such as solid or paste, of the sample for the detection. A sample for the detection may be mixed with the developer solution in advance, and the mixture may be supplied/dropped onto the sample pad (sample application portion) for development, or the sample may be supplied/dropped onto the sample pad (sample application portion) in advance, and then the developer solution may be supplied/dropped onto the sample pad (sample application portion). When the composition is used as a diluent for diluting samples, a sample diluted with the diluent may be directly supplied/dropped onto the sample pad (sample application portion), or the sample pad portion (sample application portion) may be immersed for development in the sample diluted with the diluent.

Typical examples of the sample (specimen) containing the object to be detected of the present invention include non-heated food, and food, health food, and pet food that are treated mainly for drying or sterilization treatment at a temperature at which proteins are not denatured, but the sample is not limited thereto, and livestock feed and medicines also can be used as samples (specimens).

In the present invention, a polyclonal antibody that is obtained by immunizing a rabbit or a goat with IgG (commercial product), which is an immunizing antigen, contained in porcine serum and specifically recognizes the IgG is preferably used as a detection antibody.

The polyclonal antibody of the present invention is prepared as follows:

### Preparation of polyclonal antibody

Rabbit is immunized with IgG (commercial product) contained in porcine serum in accordance with a common method to obtain antiserum. A rabbit-derived anti-porcine IgG antibody is purified from the antiserum by a procedure including dialysis with 50% saturated ammonium sulfate and then purification and collection with a DEAE column (anion-exchange column).

When the rabbit-derived anti-porcine IgG antibody is applied in this state without modifying it to immunochromatographic detection or ELISA detection, though the antibody significantly reacts with raw pork, it also reacts with a standard buffer (background) and, therefore, cannot be used as a detection antibody. Accordingly, detection of beef, lamb, and chicken was not conducted. The test results are shown in "Table 1".

In the table, "HamaPhoto" is abbreviation of "Hamamatsu Photonics densitometer".

**[Table 1]**

| (1) | | | |
|---|---|---|---|
| Use of rabbit antibody | | | |
| | HamaPhoto | Visual judgment | SN ratio |
| Buffer | 42.7 | ++ | |
| Pork | 262.5 | ++++ | 6.1 |
| Beef | - | | |
| Lamb | - | | |
| Chicken | - | | |

Accordingly, the present inventors cut the Fc region of the rabbit-derived anti-porcine IgG antibody where is highly lipophilic with, for example, cutting enzyme (e.g., papain or protease) or cut and then treat with a reducing agent (e.g., dithiothreitol), and then conducted purification and separation to obtain Fab fragment rabbit antibody, Fab' fragment rabbit antibody, or Fab'2 rabbit fragment antibody. The Fab'2 rabbit antibody is used as a detection antibody on a labeled component (for example, gold colloid) side, and detection of meat in non-heated food is conducted by immunochromatography. As a result, non-specific reaction with a standard buffer (background) can be inhibited to allow visual judgment of immunochromatographic detection of raw pork with high performance. The test results are shown in "Table 2".

**[Table 2]**

| (2) | | | |
|---|---|---|---|
| Use of Fab' fragment rabbit antibody (gold colloid side) | | | |
| | HamaPhoto | Visual judgment | SN ratio |
| Buffer | 8.9 | - | |
| Pork | 152.9 | +++ | 17.2 |
| Beef | 3.5 | - | |
| Lamb | 4.7 | - | |
| Chicken | 4.9 | - | |

Similarly, antiserum is obtained by immunizing a goat instead of rabbit with IgG (commercial product) contained in porcine serum in accordance with a common method. Purification of goat-derived anti-porcine IgG antibody from the antiserum is conducted as in the case of rabbit to obtain goat-derived anti-porcine IgG antibody.

Detection of commercially available four kinds of non-heated meat (pork, beef, lamb, and chicken) is conducted by immunochromatography using the thus obtained goat-derived anti-porcine IgG antibody. Non-specific reaction with a standard buffer (background) is not caused, a band strongly recognizing porcine IgG protein is detected, and the results by visual judgment are satisfactory. An antibody having significantly high specificity to pork, compared to those against various kinds of meat other than pork, is obtained. In immunochromatographic detection using the goat-derived anti-porcine IgG antibody as the detection antibody, since the detection sensitivity of raw pork in non-heated food is high, detection of commercially available four kinds of non-heated meat (pork, beef, lamb, and chicken) is very well even if the Fc region having high lipophilicity is not cut, compared to that in the case using rabbit-derived anti-porcine IgG antibody. The results are shown in "Table 3".

**[Table 3]**

| (3) | | | | |
|---|---|---|---|---|
| | Use of goat antibody | | | |
| | | HamaPhoto | Visual judgment | SN ratio |
| | Buffer | 3.3 | - | |
| | Pork | 361.0 | ++++ | 109.4 |
| | Beef | 1.1 | - | |
| | Lamb | 4.1 | - | |
| | Chicken | 2.4 | - | |

| | | | | |
|---|---|---|---|---|
| * HamaPhoto: Hamamatsu Photonics densitometer | | | | |

The structure of an immunochromatography apparatus or a detection kit according to the present invention will be described below.

The sample application portion is configured of a porous sheet having characteristics to rapidly absorb a sample, but hardly hold it, to allow the sample to rapidly penetrate to a reaction portion. Examples of the porous sheet include cellulose filters, glass filters, polyurethane, polyacetate, cellulose acetate, nylon, and cotton fabric. As the porous sheet of the present invention, use of glass filters is preferable. In an embodiment of the present invention, in order to inhibit non-specific reaction, the immunochromatographic reagent composition containing additives such as a buffer, a nonionic surfactant, and a protein, and according to need, a chelating agent may be held in a sample pad (sample application portion) by previously impregnating the sample pad with the immunochromatographic reagent composition and then, for example, drying it.

The labeled material-holding portion is configured so as to hold a labeled reagent composed of a labeling component and a reagent component labeled with the labeling component. Examples of the labeling component that can be used include metal colloidal particles such as gold colloidal particles and silver colloidal particles, colored latex particles obtained by dyeing synthetic polymers synthesized by (co)polymerization of various monomers, enzymes, fluorescent compounds, and other materials. The reagent component is particles or molecules having an ability of recognizing an analyte and is a polyclonal antibody or its fragment (second reagent).

The chromatographic medium is composed of a membrane carrier and a detection portion (or referred to as "reaction portion") formed on the membrane carrier. The membrane carrier is not particularly limited as long as it can absorb and transfer a sample by a capillary action. For example, the chromatographic medium is selected from the group consisting of nitrocellulose, cellulose acetate, nylon, polyether sulfone, polyvinyl alcohol, polyester, glass fiber, polyolefin, cellulose, and artificial polymers composed of mixed fibers thereof. The detection portion has a polyclonal antibody or its fragment (first reagent) immobilized on a nitrocellulose sheet.

The absorbing portion is made of a material having an ability of rapidly absorbing an excessive sample, such as a glass filter.

A backing sheet is a base material. One surface of the backing sheet is provided with adhesion by application of an adhesive or attachment of adhesive tape, and on the adhesive surface, the sample application portion, the labeled material-holding portion, the chromatographic medium (having the detection portion), and a part of or the whole absorbing portion are adhesively disposed. The backing sheet is not particularly limited as the base material as long as it is impermeable for a sample solution and for moisture by the adhesive.

Both the reagent component (first reagent) used in the detection portion (or referred to as "reaction portion") and the reagent component (second reagent) used in the labeled material-holding portion are polyclonal antibodies or their fragments, and these two kinds of antibodies are obtained by immunizing a rabbit or a goat by the same method. However, in the case of a rabbit-derived anti-porcine IgG antibody, an antigen-binding fragment obtained by, for example, enzyme fragmentation treatment is used. By doing so, it is possible to reduce manufacturing cost and maintain stable supply of the antibody while maintaining the specificity.

The polyclonal antibody and its fragment are basically prepared by a known method. The polyclonal antibody is prepared through a common method by separating a target antibody from antiserum obtained by immunizing an antibody-producing animal (rabbit or goat) with an antigen (IgG in porcine serum). For example, see the literature by Berger, et al. (J. Assoc. Off. Anal. Chem., vol. 71, no. 2, 1988).

The principle of judgment will be roughly described below:
1. A predetermined amount (usually 0.1 to 2 mL) of a sample (for example, an extraction developer solution of non-heated food containing meat) is dropped onto a sample pad. The sample applied onto the sample pad moves in the sample pad. When the sample pad is impregnated with a specific immunochromatographic reagent composition, the immunochromatographic reagent composition is dissolved in the moisture content of the sample and moves together with the sample.
2. The sample (for example, the extraction developer solution of non-heated food containing meat) or the sample dissolving the immunochromatographic reagent composition first moves to the labeled material-holding portion. During passing of the sample through the labeled material-holding portion, the labeling reagent (second reagent) being held at the labeled material-holding portion is dissolved in the moisture content of the sample to move together with the sample.
3. Then, the labeling agent dissolved in the moisture content of the sample passes through the detection portion on the chromatography medium. When the sample contains pork, the antigen in the pork specifically reacts with the antibody held, that is, immobilized on the detection portion and the labeled reagent to form a sandwich-like complex by antigen-antibody specific binding reaction, resulting in coloring in the detection portion. When the sample does not contain pork, the labeling agent dissolved in the moisture content of the sample, even if the sample passes through the detection portion on the chromatography medium, specific binding reaction does not occur. Therefore, the detection portion is not colored.
4. Lastly, the moisture content of the sample moves to the absorbing portion. Thus, the presence or absence of pork in a sample can be exactly determined. Example

The immunochromatography apparatus that is used in the present invention will be described in detail below, but it is merely an example, and the present invention is not limited thereto.

### 1. Production of a reaction portion on a chromatography medium

A goat-derived anti-porcine IgG antibody, diluted with a carbonate buffer (pH 7.5) containing 5% by weight of sucrose and 5% by weight of isopropyl alcohol to a concentration of 3.25 mg/mL, was applied onto a 25 x 2.5 cm nitrocellulose membrane (manufactured by Millipore, HF180) with an antibody coating machine (manufactured by BioDot), followed by drying at 42°C for 60 minutes and then at room temperature overnight to produce a reaction portion on the chromatography medium.

### 2. Production of labeling material solution

Goat-derived anti-porcine IgG antibody was diluted with a HEPES buffer (pH 7.5) to a concentration of 0.05 mg/mL. The diluted goat-derived anti-porcine IgG antibody (0.1 mL) was added to a gold colloidal suspension (manufactured by Tanaka Kikinzoku Kogyo K.K., 0.5 mL, average particle diameter: 60 nm), and the resulting mixture was left to stand at room temperature for 10 minutes. Subsequently, CE510 (JSR Corporation, 0.1 mL) and then 0.05 mL of 1% polyethylene glycol dissolved in a potassium phosphate solution (pH 7.5) were added thereto, followed by sufficient stirring. The mixture was left to stand at room temperature for 10 minutes and then centrifuged at 8000 x g for 15 minutes. The supernatant was removed, and a buffer (pH 7.4) containing 1% by weight of bovine serum albumin (0.1 mL) was added to the residue to obtain a labeling material solution.

### 3. Production of chromatography medium

The above-produced labeling material solution was uniformly applied onto a glass fiber pad, and the pad was dried with a vacuum dryer to obtain a detection reagent holding member. Then, the above-prepared chromatography medium, detection reagent holding member, a sample pad to be used as a portion to which a sample is applied, and an absorbing pad for absorbing the applied sample and an insoluble carrier were bonded to a base material of a backing sheet. Lastly, by bonding of a laminate seal and cutting to a width of 5 mm with a cutter, chromatography medium was obtained.

### 4. Preparation of extraction developer solution

A surfactant and sodium chloride were added to ultrapure water to obtain concentrations of 0.5% by weight and 0.2 M, respectively, and sodium azide serving as a preservative was further added thereto to obtain a concentration of 0.1%, followed by mixing.

### 5. Production of sample

To 0.5 mL of the extraction developer solution, 0.1 to 0.2 g of pork or meat derived from an animal other than hog was added, and the mixture was shaken for 30 seconds by hand.

### 6. Measurement

The presence or absence of pork in a sample was measured using the above-produced chromatography medium according to the following process. The above-produced sample containing pork was defined as a positive specimen, the above-produced sample containing meat derived from an animal other than hog was defined as a negative specimen, and the end of the chromatography medium was immersed in any of the sample solutions. After confirmation of flowing out of the gold colloid from the glass fiber pad, the immersed chromatography medium was taken out from the solution and was placed on a horizontal table. After 10 to 15 minutes, the result was visually judged.

A test method for the presence or absence of pork in test food using the above-produced chromatography apparatus (test kit) is shown in Fig. 1. The test method is conducted by the following procedures 1 to 5:
1. Add food (about 0.1 to 0.2 g) to be tested to a tube containing the above-described extraction developer solution (0.5 mL).
2. Tightly cover the tube with the lid, and shake the tube for about 30 seconds by hand.
3. Take off the lid, immerse the end of the sample application portion of the test kit in the solution, and confirm that a red solution is developed on the test kit.
4. After confirmation of infiltration of the red solution, take out the test kit, and place it on a horizontal table.
5. After 10 to 15 minutes, confirm the presence or absence of a line.

Test examples of the present invention will be described below, but they are merely examples, and the present invention is not limited thereto.

Visual judgment was performed 10 minutes after the dropping of samples, and a sample that showed a red line at the detection portion was defined as "+", a sample that showed a line more clearly in a strong (deep color) state was defined as "++", a sample that showed a line in a significantly strong state was defined as "+++", a sample did not show a red line was defined as "-", and a sample that showed a line in a weak (faint color) state was defined as (±).

### Test Example a

As non-heated test food (meat), beef containing pork at a ratio (pork/beef) of 0% by weight, 0.01 % by weight, 0.025% by weight, 1% by weight, or 5% by weight was evaluated three times. The results are shown in "Table 4".

In production Lot Nos. 1 to 3 in Table 4, each evaluation was repeated three times using goat-derived anti-porcine IgG antibody obtained by immunizing a goat with IgG in porcine serum as the detection reagent.

The results showed that pork contained in beef at a concentration of 1% by weight or more could be detected without non-specific reaction (false positive). In this Test Example, lot-to-to difference and variation in repeated tests did not occur.

**[Table 4]**

| Detection of pork in non-heated food | | | | | | |
|---|---|---|---|---|---|---|
| | | % by weight (pork/beef) | | | | |
| Production Lot. No. | N=3 | 0 | 0.01% | 0.025% | 1% | 5% |
| | 1 | - | + | ++ | +++ | +++ |
| 1 | 2 | - | + | ++ | +++ | +++ |
| | 3 | - | + | ++ | +++ | +++ |
| | 1 | - | + | ++ | +++ | +++ |
| 2 | 2 | - | + | ++ | +++ | +++ |
| | 3 | - | + | ++ | +++ | +++ |
| | 1 | - | + | ++ | +++ | +++ |
| 3 | 2 | - | + | ++ | +++ | +++ |
| | 3 | - | + | ++ | +++ | +++ |

### Test Example b

As non-heated test food (meat), chicken containing pork at a ratio (pork/chicken) of 0% by weight, 0.005% by weight, 0.025% by weight, 1% by weight, or 5% by weight was evaluated three times. The results are shown in Table 5.

In production Lot Nos. 1 to 3 in Table 5, each evaluation was repeated three times using goat-derived anti-porcine IgG antibody obtained by immunizing a goat with IgG in porcine serum as the detection reagent.

The results showed that pork contained in chicken at a concentration of 1% by weight or more could be detected without non-specific reaction (false positive). In this Test Example, lot-to-to difference and variation in repeated tests did not occur.

**[Table 5]**

| | | % by weight (pork/chicken) | | | | |
|---|---|---|---|---|---|---|
| Production Lot. No. | N=3 | 0 | 0.005% | 0.025% | 1% | 5% |
| | 1 | - | + | ++ | +++ | +++ |
| 1 | 2 | - | + | ++ | +++ | +++ |
| | 3 | - | + | ++ | +++ | +++ |
| | 1 | - | + | ++ | +++ | +++ |
| 2 | 2 | - | + | ++ | +++ | +++ |
| | 3 | - | + | ++ | +++ | +++ |
| | 1 | - | + | ++ | +++ | +++ |
| 3 | 2 | - | + | ++ | +++ | +++ |
| | 3 | - | + | ++ | +++ | +++ |

### Test Example c

The detection kit of the present invention was evaluated by measuring extraction solutions extracted from non-heated single food (100%) of pork, beef, chicken, lamb, or soybean. The measurement was repeated three times. The results are shown in Table 6.

In production Lot Nos. 1 to 3 in Table 6, each evaluation was repeated three times using goat-derived anti-porcine IgG antibody obtained by immunizing a goat with IgG in porcine serum as the detection reagent.

The results showed that only protein derived from pork was detected, whereas proteins extracted from beef, chicken, lamb, or soybean were not detected, clearly showing specificity to pork. Also in this Test Example, lot-to-to difference and variation in repeated tests did not occur.

**[Table 6]**

| Specificity confirming test (single heat-processed food) | | | | | | |
|---|---|---|---|---|---|---|
| Production Lot. No. | N=3 | Pork | Beef | Chicken | Lamb | Soybean |
| | 1 | +++ | - | - | - | - |
| 1 | 2 | +++ | - | - | - | - |
| | 3 | +++ | - | - | - | - |
| | 1 | +++ | - | - | - | - |
| 2 | 2 | +++ | - | - | - | - |
| | 3 | +++ | - | - | - | - |
| | 1 | +++ | - | - | - | - |
| 3 | 2 | +++ | - | - | - | - |
| | 3 | +++ | - | - | - | - |

### Industrial Applicability

The detection kit of the present invention is based on immunochromatography and, therefore, can be widely used in the field to which immunological assay is applied by using the antibody of the present invention immobilized on an insoluble carrier. Since the presence or absence of raw pork in non-heated food can be conveniently, rapidly, inexpensively, and specifically detected, the present invention can contribute to solve problems of patients to whom pork causes allergy reaction or of conventional wisdom depending on the reasons, for example, of not eating pork in terms of tastes or dietary cultures and also dietary habit. The immunochromatographic detection process of the present invention has industrial applicability operationally and economically superior to PCR and ELISA methods.

### Reference Signs List

- 1: Test food
- 2: Extraction developer solution
- 3: Tube
- 4: Lid
- 5: Detection chip

### Citation List

### Patent Literature

PTL 1: JP-A-2006-317226
PTL 2: JP-A-2005-164583
PTL 3: JP-A-2003-155297
PTL 4: U.S. Patent No. 6288215

### Non Patent Literature

NPL 1: Biotechnology Techniques, Vol. 11, No. 7, July 1997, pp. 533-535

## Claims

1. An immunochromatographic detection process of a protein derived from raw pork using a polyclonal antibody as a detection antibody, wherein the polyclonal antibody is obtained by immunizing a goat with porcine immunoglobulin G and said antibody specifically recognizes immunoglobulin G contained in raw pork.

2. The immunochromatographic detection process of a protein derived from raw pork according to Claim 1, **characterized in that**, the said process is performed by means of an apparatus whereas said apparatus comprises a labeled material-holding portion holding a labeled antibody that is a polyclonal antibody obtained by immunizing a goat with porcine immunoglobulin G, specifically recognizing immunoglobulin G contained in raw pork, and labeled with a labeling material, and a detection portion where a goat polyclonal antibody specifically recognizing immunoglobulin G contained in raw pork is immobilized.

3. The immunochromatographic detection process according to Claim 2, **characterized in that**, the apparatus being substantially composed of a sample application portion, a labeled material-holding portion, a chromatography medium, a detection portion, and an absorbing portion.

4. The immunochromatographic detection process for detecting a protein derived from raw pork, according to Claim 1, using a kit being substantially composed of a sample application portion, a labeled material-holding portion, a chromatography medium, a detection portion, and an absorbing portion, wherein the labeled material-holding portion holds a polyclonal antibody obtained by immunizing a goat with porcine immunoglobulin G, specifically recognizing immunoglobulin G contained in raw pork, and labeled with a labeling material; and the detection portion has a goat polyclonal antibody specifically recognizing immunoglobulin G contained in raw pork immobilized thereon.

## Patentansprüche

1. Immunochromatographisches Nachweisverfahren eines Proteins, das aus rohem Schweinefleisch stammt, unter Verwendung eines polyklonalen Antikörpers als einen Detektionsantikörper, worin der polyklonale Antikörper durch die Immunisierung einer Ziege mit porkinem Immunoglobulin G gewonnen wird und der genannte Antikörper gezielt in rohem Schweinefleisch enthaltenes Immunoglobulin G erkennt.

2. Immunochromatographisches Nachweisverfahren eines Proteins, das aus rohem Schweinefleisch stammt, gemäß Anspruch 1, **dadurch gekennzeichnet, dass** das genannte Verfahren mittels einer Vorrichtung durchgeführt wird, wobei die genannte Vorrichtung einen markierten Materialhalteabschnitt umfasst, der einen markierten Antikörper, der ein polyklonaler Antikörper ist, der durch die Immunisierung einer Ziege mit porkinem Immunoglobulin G gewonnen wird, der gezielt in rohem Schweinefleisch enthaltenes Immunoglobulin G erkennt und ein Markierungsmaterial und einen Nachweisabschnitt, wo ein polyklonaler Ziegen-Antikörper immobilisiert ist, der gezielt in rohem Schweinefleisch enthaltenes Immunoglobulin G erkennt, enthält.

3. Immunochromatographisches Nachweisverfahren, gemäß Anspruch 2, **dadurch gekennzeichnet, dass** die Vorrichtung im Wesentlichen aus einem Probenauftragungsabschnitt, einem markierten Materialhalteabschnitt, einem Chromatographiemedium, einem Nachweisabschnitt und einem Absorptionsabschnitt besteht.

4. Immunochromatographisches Nachweisverfahren eines Proteins, das aus rohem Schweinefleisch stammt, gemäß Anspruch 1, unter Verwendung eines Kits, das im Wesentlichen aus einem Probenauftragungsabschnitt, einem markierten Materialabschnitt, einem Chromatographiemedium, einem Nachweisabschnitt und einem Absorptionsabschnitt besteht, worin der markierte Materialabschnitt einen polyklonalen Antikörper enthält, der durch die Immunisierung einer Ziege mit porkinem Immunoglobulin G gewonnen wird, der gezielt in rohem Schweinefleisch enthaltenes Immunoglobulin G erkennt und der mit einem Markierungsmaterial markiert ist; und der Nachweisabschnitt einen polyklonalen Ziege-Antikörper, der gezielt in rohem Schweinefleisch enthaltenes Immunoglobulin G erkennt, hat und der in diesem immobilisiert ist.

## Revendications

1. Procédé de détection immunochromatographique d'une protéine dérivée de porc cru recourant à un anticorps polyclonal comme anticorps de détection, où l'anticorps polyclonal est obtenu par immunisation d'une chèvre avec de l'immunoglobuline G porcine et où l'anticorps reconnaît spécifiquement l'immunoglobuline G contenue dans le porc cru.

2. Procédé de détection immunochromatographique d'une protéine dérivée de porc cru selon la revendication 1, **caractérisé en ce que** ledit procédé est exécuté au moyen d'un dispositif, ledit dispositif comprenant une partie de support de matériau étiquetée supportant un anticorps étiqueté qui est un anticorps polyclonal obtenu par immunisation d'une chèvre avec de l'immunoglobuline G porcine, reconnaissant spécifiquement l'immunoglobuline G contenue dans le porc cru, et étiqueté avec un matériau d'étiquetage, et une partie de détection où un anticorps polyclonal de chèvre reconnaissant spécifiquement l'immunoglobuline G contenue dans le porc cru est immobilisé.

3. Procédé de détection immunochromatographique selon la revendication 2, **caractérisé en ce que** le dispositif est essentiellement composé d'une partie d'application d'échantillon, d'une partie de support de matériau étiquetée, d'un milieu de chromatographie, d'une partie de détection et d'une partie d'absorption.

4. Procédé de détection immunochromatographique d'une protéine dérivée de porc cru selon la revendication 1, recourant à un kit essentiellement composé d'une partie d'application d'échantillon, d'une partie de support de matériau étiquetée, d'un milieu de chromatographie, d'une partie de détection et d'une partie d'absorption, où la partie de support de matériau étiquetée supporte un anticorps polyclonal obtenu par immunisation d'une chèvre avec de l'immunoglobuline G porcine, reconnaissant spécifiquement l'immunoglobuline G contenue dans le porc cru, et étiqueté avec un matériau d'étiquetage ; et où la partie de détection comporte un anticorps polyclonal de chèvre reconnaissant spécifiquement l'immunoglobuline G contenue dans le porc cru immobilisé dans celle
